# EUROPEAN PATENT SPECIFICATION

(11) **EP 2 586 865 B1**
(45) Date of publication and mention of the grant of the patent: **21.03.2018**
(21) Application number: 11798393.2
(22) Date of filing: 23.06.2011
(51) Int. Cl.: C12N 15/63, C12N 15/12, A61K 38/00, A61P 35/00

(54) **RECOMBINANT VECTOR FOR SUPPRESSING PROLIFERATION OF HUMAN PAPILLOMAVIRUS CELL INCLUDING ADENYLATE CYCLASE ACTIVATING POLYPEPTIDE 1 (PITUITARY) GENE AND PHARMACEUTICAL COMPOSITION FOR TREATING HUMAN PAPILLOMAVIRUS**
REKOMBINANTER VEKTOR ZUR UNTERDRÜCKUNG DER ZELLVERMEHRUNG DES HUMANEN PAPILLOMAVIRUS EINSCHLIESSLICH DES ADENYLATCYCLASE-AKTIVIERENDEN POLYPEPTID-1- (HYPOPHYSEN-)GENS SOWIE PHARMAZEUTISCHE ZUSAMMENSETZUNG FÜR EINE THERAPIE GEGEN DAS HUMANE PAPILLOMAVIRUS
VECTEUR RECOMBINÉ POUR SUPPRIMER LA PROLIFÉRATION DE CELLULES DE PAPILLOMAVIRUS HUMAIN COMPRENANT LE GÈNE DU POLYPEPTIDE ACTIVANT L'ADÉNYLATE CYCLASE 1 (PITUITAIRE) ET COMPOSITION PHARMACEUTIQUE POUR TRAITER LE PAPILLOMAVIRUS HUMAIN

(30) Priority: 24.06.2010 KR 20100060196
(43) Date of publication of application: 01.05.2013
(73) Proprietor: Genomictree, Inc., Daejeon 305-510 (KR)
(72) Inventor: AN, Sung Whan, Daejeon 305-762 (KR); MOON, Young Ho, Daejeon 305-761 (KR); OH, Tae Jeong, Daejeon 305-721 (KR); LEE, Seng-Hoon, Seoul 139-745 (KR)
(74) Representative: Hiebl, Inge Elisabeth
(86) International application number: PCT/KR2011/004594
(87) International publication number: WO 2011/162558

(56) References cited:
- WO-A1-99/51762
- WO-A2-2004/108899
- WO-A2-2005/085861
- WO-A2-2006/099590
- WO-A2-2011/020119
- WO-A2-2012/112792
- KR-A- 20070 088 298
- US-A1- 2004 038 888
- US-A1- 2006 211 009
- RONG-JIE YU ET AL.: 'Intein-mediated Rapid Purification of Recombinant Human Pituitary Adenylate Cyclase Activating Polypeptide.' ACTA BIOCHIMICA ET BIOPHYSICA SINICA vol. 36, no. 11, 2004, pages 759 - 766, XP055096537
- DATABASE GENBANK [Online] 17 July 2006 'Adenylate cyclase activating polypeptide 1 (pituitary) [Homo sapiens]', XP003033136 Retrieved from NCBI Database accession no. AAI01804

## Description

### TECHNICAL FIELD

The present invention relates to a pharmaceutical composition for use in the treatment of cervical cancer comprising a recombinant vector comprising a gene of SEQ ID NO: 1 that encodes Adenylate Cyclase Activating Polypeptide 1 (pituitary) (*ADCYAP1;* NM_001099733) or a gene that encodes a fragment of ADCYAP1 comprising a sequence of SEQ ID NO: 9 or 10 as an active ingredient, or adenylate cyclase activating polypeptide 1 (pituitary) (ADCYAP1; NM_001099733) of SEQ ID NO: 2, or a fragment of ADCYAP1 comprising a sequence of SEQ ID NO: 9 or 10 as an active ingredient. More particularly the present invention relates to the pharmaceutical composition for use in the treatment of cervical cancer comprising a recombinant vector containing either a adenylate cyclase activating polypeptide 1 (pituitary) (*ADCYAP1*)-encoding gene whose promoter region is methylated specifically in cervical cancer cells to significantly inhibit the expression of the gene, or a fragment of the gene, in which the recombinant vector inhibits the proliferation of cervical cancer cells when it is introduced into the cervical cancer cells.

### BACKGROUND ART

Oncogenes, tumor suppressor genes and apoptosis regulating genes in normal cells are so controlled complementarily that a growth and maintenance of the cells are balanced. However, when the abnormal cell growth and proliferation caused by various factors is repeated, cancer occurs. The major causes of carcinogenesis are the abnormalities of genes in cells. Such genetic abnormalities typically include genetic variations and epigenetic variations.

CpG islands are found in many regions, including the coding sequence upstream of the regulatory region including the promoter region of a specific gene, the coding region (e.g., exon region), and the region downstream of the coding region (e.g., enhancer region and intron). DNA methylation that is one of epigenetic variations occurs mainly at the cytosine of CpG islands in the promoter region of a specific gene, and the methylation of CpG islands in the promoter that regulates gene expression inhibits the expression of the target gene. In particular, it was reported that tumor suppressor genes, DNA repair genes, cell cycle-regulating genes and the like in various cancer cells are hyper-methylated, suggesting that the expression of these genes is silenced. Also, it was found that the expression of oncogenes is induced by hypo-methylation. Particularly, hyper-methylation and hypo-methylation are found in the initial stage of carcinogenesis. Accordingly, methylation of CpG islands in the promoter region of a specific gene in cancer cells inhibits or silences the expression of the gene, and ultimately inhibits or inactivates the activity of the gene in the cells. Thus, it generally appears that a gene which is methylated in cancer cells is highly likely to be a tumor suppressor gene (Baylin et al., Adv. Cancer Res., 72:141, 1998; Jones and Laird, Nat. Genet., 21: 163, 1999).

For treatment of cancer which is the first leading cause of death of humans, extensive studies have been conducted. Cancer treatment methods known to date include surgery, radiotherapy, anticancer chemotherapy, immunotherapy and gene therapy. Such studies on the treatment of cancer have suggested the need for a new therapeutic method of introducing and expressing a cancer therapeutic gene selectively in cancer cells and cells in cancer tissue.

With respect to recent gene therapy, there have been studies on methods for discovering a new target gene, efficiently introducing the discovered target gene into a target cell, inducing the long-term expression of the introduced gene and increasing the efficiency of delivery of the gene in clinical therapy.

Known gene therapy methods of using a gene or its protein to treat cancer include a method that uses a tumor inhibitor containing, as an active ingredient, a p43 protein comprising a specific nucleotide sequence that induces cytokine production to increase an immune response and inhibits angiogenesis (Korean Patent Laid-Open Publication No. 2001-0112108), an anticancer agent containing mycolactone that kills cancer cells, an antisense Rb nucleotide that inhibits the expression of retinoblastoma protein, and an anticancer agent containing mycolactone and the antisense Rb nucleotide (Korean Patent Laid-Open Publication No. 2002-0040521). In addition, other known methods include a method of inhibiting cancer using an anticancer composition comprising a recombinant vector that expresses importin-a gene and recombinant p53 gene (Korean Patent Laid-Open Publication No. 10-2005-0098800), and a composition for treatment and diagnosis of prostate cancer, which contains a prostate-specific protein or a gene encoding the same (US Patent No. 6,329,505).

In addition, genes or their proteins have been frequently used for cancer treatment, and studies thereon have been continued. At present, studies on the discovery of genes deficient in cancer patients, or cancer-inducing genes and anticancer genes in parallel with the human genome project are in progress. Particularly, there is a very urgent need for the discovery of new cancer suppressor genes that can be more effectively used for gene therapy of cancer.

The present inventors previously have found that the expression of a gene in cancer tissue cells is inhibited by DNA methylation at CpG islands in the promoter region of the gene (Korean Patent Laid-Open Publication No. 10-2007-0109811).

Accordingly, the present inventors have made extensive efforts to develop an agent for treating cervical cancer using a gene, and as a result, have found that the promoter region of a gene that encodes adenylate cyclase activating polypeptide 1 (pituitary) (*ADCYAP1*) is methylated specifically in a cervical cancer cell line, tissue and scrape to inhibit the expression of the gene, and also have found that, when a recombinant vector containing a gene that encodes adenylate cyclase activating polypeptide 1 (pituitary) (*ADCYAP1*) is introduced into cervical cancer cells, it shows the effect of inhibiting the proliferation of the cervical cancer cells, thereby completing the present invention.

### DISCLOSURE OF INVENTION

It is a main object of the present invention to provide a pharmaceutical composition, wherein the composition comprises
(i) a recombinant vector comprising a gene of SEQ ID NO: 1 that encodes adenylate cyclase activating polypeptide 1 (pituitary) (ADCYAP1; NM_001099733), or a gene that encodes a fragment of ADCYAP1 comprising a sequence of SEQ ID NO: 9 or 10 as an active ingredient, or
(ii) adenylate cyclase activating polypeptide 1 (pituitary) (ADCYAP1; NM_001099733) of SEQ ID NO: 2, or a fragment of ADCYAP1 comprising a sequence of SEQ ID NO: 9 or 10 as an active ingredient, for use in the treatment of cervical cancer.

Other features and embodiments of the present invention will be more apparent from the following detailed descriptions and the appended claims.

### BRIEF DESCRIPTION OF THE DRAWINGS

FIG. 1 shows a process in which the *ADCYAP1* gene, whose expression in cervical cancer cells is down-regulated, is screened by measuring methylation using the analysis of gene expression, in-silico analysis and pyrosequencing in cervical cancer tissue and cell line.
FIG. 2 shows the expression level of *ADCYAP1* gene in cervical tissue (FIG. 2A) and the increase in expression of *ADCYAP1* gene after treatment of cervical cancer cells with DAC (FIG. 2B).
FIG. 3 shows the methylation levels of *ADCYAP1* gene in cervical cancer cell lines (FIG. 3A) and cervical cancer tissue (FIG. 3B).
FIG. 4 shows the results of measuring the methylation levels of *ADCYAP1* gene in normal and cervical cancer scrapes (FIG. 4A) and the results of measuring the sensitivity and specificity of *ADCYAP1* gene to cervical cancer diagnosis by ROC curve analysis (FIG. 4B).
FIG. 5 shows the results of measuring the effect of *ADCYAP1* gene on the proliferation of a cervical cancer cell line (FIG. 5A: the results of microscopic observation of whether a HeLa cervical cancer cell line introduced with a p*ADCYAP1* recombinant vector did proliferate; FIG. 5B: the results of measuring the number of dead cells by ELISA).
FIG. 6 shows the results of WST assay (FIG. 6A) and optical microscopic observation (FIG. 6B) of a cervical cancer cell line treated with a peptide fragment of the *ADCYAP1* protein.

### BEST MODE FOR CARRYING OUT THE INVENTION

Unless defined otherwise, all technical and scientific terms used herein have the same meaning as commonly understood by one of ordinary skill in the art to which the invention pertains. Generally, the nomenclature used herein and the experiment methods which will be described later are those well known and commonly employed in the art.

In one aspect, the present invention is directed to a pharmaceutical composition comprising a recombinant vector for inhibiting proliferation of cervical cancer cells, which contains either a gene that encodes adenylate cyclase activating polypeptide 1 (pituitary) (*ADCYAP1* ; NM_001099733), or a gene that encodes a fragment of ADCYAP1 comprising a sequence of SEQ ID NO: 9 or 10..

In the present invention, the *ADCYAP1* protein may be represented by SEQ ID NO: 2 (GenBank NP_001093203), and the adenylate cyclase activating polypeptide 1 (pituitary) (*ADCYAP1*)-encoding gene in the recombinant vector may be a human cDNA represented by SEQ ID NO: 1.

Presently described are genes whose promoters are methylated specifically in cervical cancer cells. The relationship between the hyper-methylation of a gene that encodes adenylate cyclase activating polypeptide 1 (pituitary) (*ADCYAP1*) and the expression level of the gene was analyzed, and a recombinant vector containing the gene was constructed and introduced into cervical cancer cells to determine whether it can inhibit the proliferation of cervical cancer cells, thereby determining whether the recombinant vector or *ADCYAP1* can be used for the treatment of cervical cancer.

Further described is a fragment of the gene that encodes ADCYAP1, which preferably has 30-527 nucleotides of the gene that encodes ADCYAP1.

In one Example of the present disclosure, in order to examine the expression of the *ADCYAP1* gene in cervical cancer cells, microarray hybridization was performed. Specifically, the expression levels of *ADCYAP1* gene in total RNAs isolated from tumor tissues of cervical cancer patients and normal tissues near the tumor tissues, respectively, were measured by an indirect comparison method. In addition, in order to examine whether the expression of the gene is regulated by the methylation of the promoter of the gene, the gene was treated with 5-aza-2'-deoxycytidine (DAC) that is a demethylating agent, and as a result, the expression of the gene was increased by treatment with DAC, suggesting that the expression of the gene was inhibited by the methylation of the promoter.

Also described is a recombinant vector containing the *ADCYAP1* gene, which was introduced into cervical cancer cells, and as a result, it was found that the *ADCYAP1* gene of SEQ ID NO: 1, the promoter region of which was not methylated, was expressed in the cervical cancer cells, and thus the proliferation of the cervical cancer cells was inhibited. Furthermore, it was found that, when cervical cancer cells are demethylated by treatment with a demethylating agent, the *ADCYAP1* gene of SEQ ID NO: 1, the promoter region of which is not methylated, or a gene having sequence similarity thereto, is expressed, whereby the proliferation of the cervical cancer cells can be inhibited. The demethylating agent having such an effect can be used as an anticancer substance for inhibiting the proliferation of cervical cancer cells. It is further described that an anticancer substance can be selected by treating the *ADCYAP1* gene, the promoter region of which was methylated, with various anticancer candidate substances, examining the degree of demethylation of the gene, and selecting a substance showing a high degree of demethylation among the candidate substances.

In another Example of the present disclosure, whether the promoter of the *ADCYAP1* gene was methylated in cervical cancer cells was examined by pyrosequencing. As a result, it could be seen that the promoter of the *ADCYAP1* gene was methylated in cervical cancer cells.

In still another Example of the present disclosure, in order to deliver the *ADCYAP1* gene into cervical cancer cells, the *ADCYAP1* gene was inserted into the pcDNA™3 vector (Invitrogen), thereby constructing an *ADCYAP1* gene expression vector (p*ADCYAP1*) capable of expressing the *ADCYAP1* gene. In addition, in order to examine whether cervical cancer cells proliferate when the *ADCYAP1* gene expression vector (p*ADCYAP1*) is introduced into the cervical cancer cells, 0.1-2 µg of the p*ADCYAP1* expression vector per 10⁵ cells and 1-20 µg of liposome per 10⁵ cells were introduced into cervical cancer cells which were then cultured. As a result, it was found that the *ADCYAP1* gene was expressed with high efficiency due to the introduction of the *ADCYAP1* gene expression vector (p*ADCYAP1*), thereby inhibiting the proliferation of the cervical cancer cells.

The *ADCYAP1* gene can be isolated from human tissue or synthesized according any DNA synthesis method known in the art. In the present invention, among the 531-bp nucleotide sequence of *Homo sapiens* cDNA, the *ADCYAP1* gene (GenBank NM_001099733; SEQ ID NO: 1) was used.

In the present invention, the *ADCYAP1* protein represented by SEQ ID NO: 2 may preferably be used. The *ADCYAP1* protein can be prepared by culturing microorganisms transformed with a recombinant vector containing an *ADCYAP1* protein-encoding gene to express the protein and recovering the expressed protein by a conventional method.

A pcDNA3 vector was used to introduce the *ADCYAP1* gene into cervical cancer cells and various vectors may be used depending on the purpose of expression. In addition, the size, nucleotide sequence and the like of the gene that is inserted into the foreign gene insertion region of the expression vector can be variously changed by a known method.

Further described is a constructed recombinant vector, which can be introduced into cervical cancer cells by a conventional method. Methods for introducing the recombinant vector into cells include any method for introducing nucleic acid into cells, and introduction of the recombinant vector can be performed using a known suitable technique selected depending on a host cell. Examples of the methods for introducing the recombinant vector into cells include electroporation, calcium phosphate precipitation, calcium chloride precipitation, retrovirus infection, microinjection, PEG, a cationic liposome method, a lithium acetate-DMSO method and so on.

In one aspect, the present invention is directed to a pharmaceutical composition for treating cervical cancer, which contains, as an active ingredient, a recombinant vector containing a gene that encodes *ADCYAP1* or a fragment of the gene; the *ADCYAP1* protein; or a fragment of the *ADCYAP1* protein, as defined in the present claims.

The present disclosure includes a fragment of the *ADCYAP1* protein which has 10-175 amino acid residues of the amino acid sequence of the *ADCYAP1* protein.

In the present invention, an *ADCYAP1* protein peptide fragment comprising a nucleotide sequence of SEQ ID NO: 9 or 10 was introduced into the cervical cancer cell line Hela, and as a result, it was found that the peptide fragment of the *ADCYAP1* protein induced the apoptosis of the cervical cancer cells and inhibited the growth of the cervical cancer cells.

The pharmaceutical composition of the present invention may be formulated or used in combination with one or more agents selected from among antihistamine agents, inflammatory agents, anticancer agents and antibiotics.

The pharmaceutical composition of the present invention may be used in the form of a pharmaceutically acceptable salt thereof and may be used alone or in combination with other pharmaceutically active compounds as well as by binding to the other pharmaceutically active compounds.

The pharmaceutical composition according to the present invention can be formulated according to a conventional method. For example, it may be formulated in the form of powders, granules, tablets, capsules, suspensions, emulsions, syrups, aerosols, agents for external applications, suppositories, and sterile injection solutions. Carriers, excipients and diluents that can be contained in the composition according to the present invention include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate, and mineral oil.

A pharmaceutical composition comprising (i) the recombinant vector comprising a gene of SEQ ID NO: 1 that encodes adenylate cyclase activating polypeptide 1 (pituitary) (ADCYAP1; NM_001099733), or a gene that encodes a fragment of ADCYAP1 comprising a sequence of SEQ ID NO: 9 or 10 as an active ingredient, or
(ii) the adenylate cyclase activating polypeptide 1 (pituitary) (ADCYAP1; NM_001099733) of SEQ ID NO: 2, or a fragment of ADCYAP1 comprising a sequence of SEQ ID NO: 9 or 10 as an active ingredient is formulated using diluents or excipients, such as fillers, extenders, binders, wetting agents, disintegrants or surfactants, which are commonly used. Solid Formulations for oral administration include tablets, pills, powders, granules, capsules, etc. Such solid Formulations are prepared by mixing the extract of present invention with at least one excipient, such as starch, calcium carbonate, sucrose, lactose, gelatin, etc. In addition to simple expedients, lubricants such as magnesium stearate, talc, etc. may also be added. Liquid Formulations for oral administration, such as suspensions, internal solutions, emulsions, syrups, etc., may include simple diluents, e.g., water and liquid paraffin, as well as various excipients, e.g., wetting agents, sweeteners, aromatics, preservatives, etc. Formulations for parenteral administration include sterilized aqueous solutions, non-aqueous solvents, suspensions, emulsions, lyophilized agents, suppositories, etc. Non-aqueous solvents and suspensions may be prepared using propylene glycol, polyethylene glycol, vegetable oils such as olive oil, or injectable esters such as ethyloleate. As a base for suppositories, Witepsol, Macrogol, Tween 61, cacao fat, laurin fat, glycerogelatin, etc. may be used.

The preferred dosage of the pharmaceutical composition of the present invention can be suitably selected depending on various factors, including the patient's condition and weight, the severity of disease, the type of drug, the route and period of administration, and can be suitably determined by a person skilled in the art. In order to achieve the desired effects, however, the extract of the present invention may be administered at a daily dose of from 0. 0001 to 100 mg/kg, and preferably 0.001 to 100 mg/kg. The extract may be administered in a single dose per day or in multiple doses per day. The dosage is not intended to limit the present invention in any way.

The pharmaceutical composition of the present invention may be administered by various routes to mammals, including rats, mice, livestock and humans. All routes of administration can be contemplated and include, for example, oral, rectal, intravenous, intramuscular, subcutaneous, intrauterine, or intracerebrovascular injections.

A compound or a pharmaceutically acceptable salt thereof which is contained in the composition of the present invention may be used as a main component, additive or supplement for various functional foods and health supplement foods.

In still another aspect, the present disclosure is directed to a gene therapeutic agent for cervical cancer wherein a gene that encodes adenylate cyclase activating polypeptide 1 (pituitary) (*ADCYAP1;* NM_001099733), is introduced in an *in vivo* delivery vehicle.

As used herein, the term "gene therapeutic agent" refers to a therapeutic agent that is used in gene therapy for treating disease by gene delivery and expression. The gene therapy has various advantages, including the accurate delivery of a genetic factor into a disease site, the complete degradation of the genetic factor *in vivo,* the absence of toxicity and immune antigenicity, and long-term stable expression of the genetic factor.

Techniques for delivering a target gene include a viral vector-based transfer method that uses virus as a carrier, a non-viral delivery method that uses synthetic phospholipid or a synthetic cationic polymer, and a physical method, such as electroporation of introducing a gene by applying transient electrical stimulation to a cell membrane. Among the gene delivery techniques, the viral vector-based transfer method is considered to be preferable for the gene therapy because the transfer of a genetic factor can be efficiently made with a vector with the loss of a portion or whole of replicative ability, which has a gene substituted with a therapeutic gene. Examples of virus used as the virus carrier or vector include RNA virus vectors (retrovirus vectors, lentivirus vector, etc.), and DNA virus vectors (adenovirus vectors, adeno-associated virus vectors, etc.). In addition, its examples include herpes simplex viral vectors, alpha viral vectors, etc.

The *in vivo* delivery vehicle serves to deliver a target gene *in vivo* and maybe a virus carrier, synthetic phospholipid or a synthetic cationic polymer, which is used in technologies for delivering a target gene.

The present disclosure is further directed to a method for screening an anticancer substance for treating cervical cancer, the method comprising the steps of:
(a) treating a sample, which contains an ADCYAP1 gene whose promoter region was methylated, with an anticancer candidate substance; and
(b) selecting the candidate substance as an anticancer substance if the candidate substance demethylates the ADCYAP1 gene whose promoter region was methylated.

As is further described, the sample containing the *ADCYAP1* gene whose promoter region was methylated can be prepared using a cervical cancer cell line, tissue or scrape. The step of treating the sample with the candidate substance is performed by bringing the anticancer candidate substance and the sample into contact with each other by a conventional method.

Examples of candidate substances that demethylate the *ADCYAP1* gene whose promoter region was methylated include 5-aza-2'-deoxycytidine (DAC), the histone deacetylase inhibitor trichostatin A, trapoxin, oxamflatin, N-hydroxy-N'-phenyloctanediamide (SAHA), CBHA (m-carboxycinnamic acid bis-hydroxamide), butyric acid, phenylbutyric acid, HC toxin, depsipeptide, N-acetyldinaline, MS-275, and the like.

The step of selecting a candidate substance, which demethylates the *ADCYAP1* gene whose promoter region was methylated, as an anticancer substance, can be performed by comparing the sequence of the *ADCYAP1* gene between before and after treatment with the anticancer candidate substance.

"Indirect comparison" is performed by evaluating the level of a nucleic acid isolated from a first source and the level of a nucleic acid isolated from a second source, by the use of a reference probe which is hybridized to each of the nucleic acids isolated from the first and second sources, and comparing the results of the evaluation, thereby determining the relative amounts of nucleic acids in the sample without direct competitive binding to the reference probes. As a result, it could be seen that the expression of genes in tumor tissue was down-regulated and the expression of the *ADCYAP1* gene was also down-regulated.

As used herein, the term "vector" refers to a DNA construct containing a DNA sequence which is operably linked to a suitable control sequence capable of effecting the expression of the DNA in a suitable host. The vector may be a plasmid, a phage particle, or simply a potential genomic insert. Once transformed into a suitable host, the vector may replicate and function independently of the host genome, or may, in some instances, integrate into the genome itself. As used herein, the terms "plasmid" and "vector" are sometimes used interchangeably, because the plasmid is the most commonly used form of vector at present. For the purpose of the present invention, the plasmid vector is preferably used. A typical plasmid vector which can be used for this purpose contains the following: (a) a replication origin by which replication occurs efficiently such that several hundred plasmid vectors per host cell are created; (b) an antibiotic-resistant gene by which host cells transformed with the plasmid vector can be selected; and (c) restriction enzyme digestion sites into which foreign DNA fragments can be inserted. Even if suitable restriction enzyme digestion sites are not present in the vector, the use of a conventional synthetic oligonucleotide adaptor and linker enables easy ligation between the vector and the foreign DNA fragments.

As used herein, the term "transformation" refers to introducing DNA into a host cell so that the DNA is replicable, either as a chromosomal integrant or as an extrachromosomal element. In other words, it refers to introducing external DNA into a cell to cause a genetic change. Generally, transformation methods include electroporation, calcium phosphate (CaPO₄) precipitation, calcium chloride (CaCl₂) precipitation, microinjection, a lithium acetate-DMSO method and so on. A host cell in which an introduced DNA is highly efficiently introduced and is expressed with high efficiency is generally used as a transformed recombinant microorganism. All microbial cells, including prokaryotic and eukaryotic cells, are used. Specifically, bacteria, yeasts, fungi and the like may be used.

### EXAMPLES

Hereinafter, the present invention will be described in further detail with reference to examples. It will be obvious to a person having ordinary skill in the art that these examples are for illustrative purposes only..

In the following Examples, only a pcDNA3 vector was particularly used as an expression vector, and the use of viral vectors such as an adeno-associated virus (AAV) as an expression vector was not described. However, it will be obvious to those skilled in the art that a known viral vector that is a gene vehicle may be used to deliver a gene for treating cervical cancer *in vivo.*

In addition, in the following Examples, it was found that, when a recombinant vector containing a gene that encodes adenylate cyclase activating polypeptide 1 (pituitary) (*ADCYAP1*) was introduced, the proliferation of cervical cancer cells was inhibited by the over-expression of the *ADCYAP1* gene. However, it will be obvious to those skilled in the art that, when the *ADCYAP1* gene whose promoter region was methylated is treated with a demethylating agent, the gene whose expression was inhibited is reactivated to inhibit the proliferation of cervical cancer cells.

### Example 1: Measurement of expression of gene that encodes adenylate cyclase activating polypeptide 1 (pituitary) (ADCYAP1) in cervical cancer tissue and cervical cancer cell line

In order to examine the expression of a gene that encodes adenylate cyclase activating polypeptide 1 (pituitary) (*ADCYAP1*) in cervical cancer tissue or a cervical cancer cell line, microarray hybridization was performed according to a standard protocol(Schena et al., Science, 270:467, 1995).

Tumor tissue from a cervical cancer patient and normal tissue from a myoma patient who underwent hysterectomy were obtained from the Cancer Metastasis Research Center, Yonsei University, and total RNA was isolated from the tissues. To indirectly compare gene expression levels between the normal tissue and the tumor tissue, RNA for standard comparison (indirect comparison) was constructed.

To construct RNA for standard comparison, total RNA was isolated from the cervical cancer cell line A549 (Korean Cell Line Bank: KCLB 10185), the stomach cancer cell line AGS (KCLB 21739), the renal cancer cell line Caki-2 (KCLB 30047), the colon cancer cell line HCT116 (KCLB 10247), the cervical cancer cell line Hela (KCLB 10002), the blood cancer cell lines HK-60 (KCLB 10240) and HT1080 (KCLB 10121), the breast cancer cell line MDA-MB231 (KCLB 30026), the liver cancer cell line SK-hep1 (KCLB 30052), the T cell-derived cell line Molt-4 (KCLB 21582) and the brain cancer cell line U-87MG (KCLB 30014) using Tri reagent (Sigma, USA). To construct RNA for standard comparison, the total RNAs isolated from the 11 cell lines were mixed in the same amount and used as an internal control.

To compare relative gene expression levels between the normal tissue and the tumor tissue, the RNAs isolated from the normal and tumor tissues were compared indirectly with the RNA for standard comparison. Specifically, 100 µg of total RNA was labeled with Cy3-dUTP or Cy5-dUTP, the RNA for standard comparison was labeled with Cy3, and the RNAs isolated from the tissues were labeled with Cy5. The two cDNAs labeled with Cy3 and Cy5 were purified using a PCR purification kit (Qiagen, Germany). The purified cDNAs were mixed and concentrated to a final volume of 27 µℓ using Microcon YM-30 (Millipore Co., USA).

80 µℓ of a hybridization reaction solution (27 µℓ of labeled cDNA target, 20 µℓ of 20X SSC, 8 µℓ of 1% SDS, 24 µℓ of formamide (Sigma, USA) and 20 µℓ of human Cot1 DNA (Invitrogen, USA)) was heated at 100 °C for 2 minutes, and then immediately, was hybridized to a human 35K oligonucleotide microarray (GenomicTree, Inc., Korea) in a humidity-controlled HybChamber X (GenomicTree, Inc., Korea) at 42 °C for 12-16 hours. The hybridized microarray slide was scanned using Axon 4000B (Axon Instrument Inc., USA). The signal and background fluorescence intensities were measured for each probe by averaging the intensities of all pixels in a target using the GenePix Pro 4.0 software (Axon Instrument Inc., USA). Spots showing clear abnormality were excluded from the analysis. All data were subjected to normalization, statistical analysis and cluster analysis using GeneSpring 7.3 (Agilent, USA).

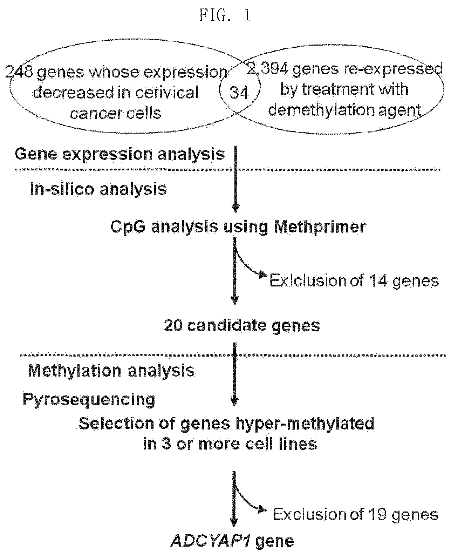

In addition, to determine the relative difference in gene expression level between the normal and tumor tissues, statistical analysis (ANOVA, p<0.01) for indirect comparison was performed. As a result, it was shown that 282 genes in the tumor tissue were all down-regulated compared to the normal tissue (see FIG. 1).

In order to examine whether the expression of the genes is regulated by the methylation of the gene promoters, the cervical cancer cell lines C33A (ATCC HTB-31), SiHa (KCLB 30035), HeLa (KCLB 10002) and Caski (KCLB 21550) were treated with 200 nM of the demethylating agent 5-aza-2'-deoxycytidine (DAC, Sigma, USA) for 3 days. The cell lines treated with DAC and the cell lines not treated with DAC were treated with Tri reagent, and total RNA was isolated from the cell lines.

In order to examine the change in gene expression caused by DAC treatment, the level of transcription was compared directly between the non-treated cells and the treated cell lines. Each of the cervical cancer cell lines was cultured in RPMI medium (GIBCO/BRL, Grand Island, NY) containing 10% FBS, 100 units/Mℓ of penicillin and 100 µg/Mℓ of streptomycin under the conditions of 37 °C and 5% CO₂, and then the expressions of genes in the DAC-treated group were measured in comparison with the control group not treated with DAC. As a result, it could be seen that the expressions of a total of 2,428 genes in the DAC-treated group increased.

The list of the 282 genes whose expression was down-regulated in cervical cancer tissue was compared with the list of the 2,428 re-expressed genes, and 34 common genes which were common between the two lists were selected as gene candidates whose expression is down-regulated by methylation in cervical cancer cells (see FIG. 1).

To find the presence of CpG islands in the promoter regions of the 34 genes, MethPrimer (http://itsa.ucsf.edu/∼urolab/methprimer/index1.html) was used. Among the 34 genes, 14 genes had no CpG island and were excluded from the common gene list (see FIG. 1).

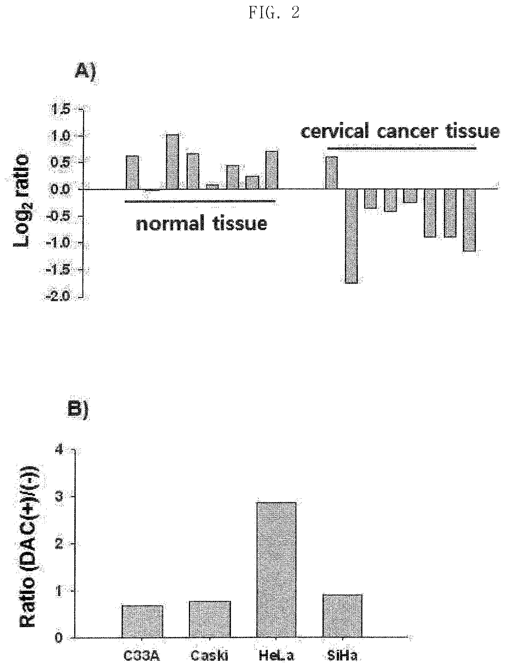

To find a gene which is expressed by methylation among the 20 genes, the degrees of methylation of the genes in 4 cervical cancer lines (C33A (ATCC HTB-31), SiHa (KCLB 30035), HeLa (KCLB 10002) and Caski (KCLB 21550)) were measured by pyrosequencing. As a result, the *ADCYAP1* showing hyper-methylation in 3 or more of the cervical cancer cell lines was selected as a gene whose cancer suppression function is to be examined (see FIG. 1). FIG. 2 shows the results of DNA microarray analysis, which indicate that the *ADCYAP1* gene was down-expressed in cervical cancer cells and re-expressed in the cervical cancer cell lines treated with DAC.

### Example 2: Measurement of methylation of gene that encodes adenylate cyclase activating polypeptide 1 (pituitary) in cervical cancer cell line

In order to examine whether the re-expression of the *ADCYAP1* gene in the cervical cancer cell lines, shown in Example 1, is attributable to demethylation, the methylation degree of the gene in the cervical cancer cell lines was measured.

Specifically, to convert unmethylated cytosine to uracil by treatment with bisulfite, total gDNA was isolated to the cervical cancer cell lines C33A (ATCC HTB-31), SiHa (KCLB 30035), HeLa (KCLB 10002) and Caski (KCLB 21550), and 200 ng of the gDNA was treated with bisulfite using an EZ DNA methylation-gold kit (Zymo Research, USA), after which it was eluted with 20 µℓ of sterile distilled water and used in pyrosequencing.

PCR and sequencing primers for performing pyrosequencing of the *ADCYAP1* gene were designed using the PSQ assay design program (Biotage, USA). PCR and sequencing primers for measuring the methylation of the *ADCYAP1* gene are shown in Table 1 below.

**Table 1**

| PCR and sequencing primers for pyrosequencing of *ADCYAP1* gene | | | | |
|---|---|---|---|---|
| Primers | Nucleotide sequence (5' → 3') | position^{a} | SEQ ID NOs. | Size of the amplified product |
| forward | gggtggatt tayggttatt ttgt | -1,210 | 3 | 345 bp |
| reverse | Biotin-aaattttccctccttaccc | -884 | 4 | |
| sequencing | g tttttgttt agatatta | -1,011 | 5 | |

| | | | | |
|---|---|---|---|---|
| position^{a}: position from transition start point (+1): nucleotide y: C or T | | | | |

The nucleotide sequence amplified with the primers of Table 1 after bisulfite treatment, and the analyzed nucleotide sequence for the sequencing primer and the measurement of methylation are as shown in SEQ ID NO: 6 below. The underlined nucleotide sequence is a nucleotide sequence analyzed to measure the methylation of the CpG island region, and the portion indicated by "y (C or T)" indicates cytosine of the CpG island region.

20 ng of the gDNA treated with bisulfite was amplified by PCR. Specifically, a PCR reaction solution (20 ng of DNA treated with bisulfite, 5 µℓ of 10X PCR buffer (Enzynomics, Korea), 5 units of Taq polymerase (Enzynomics, Korea), 4 µℓ of 2.5mM dNTP (Solgent, Korea), 2 µℓ of 10 pmole/µℓ of PCR primers) was treated at 95 °C for 5 minutes, and then subjected to PCR under the following conditions: 45 cycles of 40 sec at 95 °C, 45 sec at 60 °C and 40 sec at 72 °C, followed by 5 min at 72 °C. The amplification of the PCR product was confirmed by electrophoresis using 2.0% agarose gel.

The amplified PCR product was subjected to pyrosequencing using PyroGold reagent (Biotage, USA) by a PSQ96MA system (Biotage, USA). After pyrosequencing, the degree of methylation was determined by calculating the methylation index. The methylation index was calculated by determining the average rate of cytosine binding to each CpG island. In the case of the *ADCYAP1* gene, the methylation degree of 4 CpG island regions was measured.

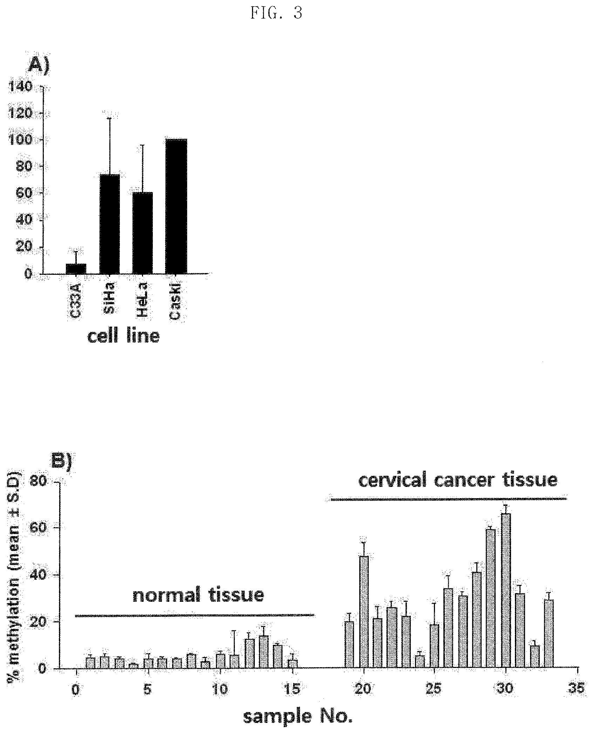

As a result, the promoter region of the *ADCYAP1* gene was not methylated (less than 10%) in the C33A (ATCC HTB-31) cell line and was hyper-methylated (50% or more) in the SiHa (KCLB 30035), HeLa (KCLB 10002) and Caski (KCLB 21550) cell lines (see FIG. 3A).

### Example 3: Measurement of methylation of gene that encodes adenylate cyclase activating polypeptide 1 (pituitary) in cervical cancer tissue

In order to examine whether the decrease in the expression of the *ADCYAP1* gene in cervical cancer tissue is attributable to methylation, the degree of methylation was quantitatively analyzed by pyrosequencing.

Normal cervical tissues (Department of Obstetrics and Gynecology, Chungnam National University Hospital) and surgical tissues from cancer patients (Department of Obstetrics and Gynecology, Chungnam National University Hospital) were obtained, and the methylation degrees of the *ADCYAP1* gene in the tumor tissues and the normal tissues were measured. Genomic DNA was isolated from each of the tumor tissues and the normal tissues using a QIAamp DNA Mini kit (QIAGEN, USA), and 200 ng of the isolated gDNA was treated with bisulfite using an EZ DNA methylation-Gold kit (Zymo Research, USA). The DNA treated with bisulfite was eluted with 20 µℓ of sterile distilled water and used in pyrosequencing.

20 ng of the gDNA treated with bisulfite was amplified by PCR. A PCR reaction solution (20 ng of DNA treated with bisulfite, 5 µℓ of 10X PCR buffer (Enzynomics, Korea), 5 units of Taq polymerase (Enzynomics, Korea), 4 µℓ of 2.5 mM dNTP (Solgent, Korea), and 2 µℓ of 10 pmole/µℓ of PCR primers) was treated at 95 °C for 5 minutes, and then subjected to PCR under the following conditions: 45 cycles of 40 sec at 95 °C, 45 sec at 60 °C and 40 sec at 72 °C, followed by 5 min at 72 °C. The amplification of the PCR product was confirmed by electrophoresis using 2.0% agarose gel.

The PCR product was subjected to pyrosequencing using PyroGold reagent by a PSQ96MA system (Biotage, USA). After pyrosequencing, the degree of methylation was determined by calculating the methylation index. The methylation index was calculated by determining the average rate of cytosine binding to each CpG island.

As a result, it was shown that the *ADCYAP1* gene was methylated at very low levels (less than 20%) in the normal tissues, but methylated at very high levels (20% or higher) in most of the cancer tissues (see FIG. 3B). Such results indicate that the decrease in the expression of the *ADCYAP1* gene in cervical cancer tissue is attributable to methylation.

### Example 4: Measurement of methylation of gene that encodes adenylate cyclase activating polypeptide 1 (pituitary) in cervical cancer scrape

In order to examine whether the decrease in the expression of the *ADCYAP1* gene in cervical cancer scrape is attributable to methylation, the degree of methylation was quantitatively analyzed by pyrosequencing.

Normal cervical scrapes (Department of Obstetrics and Gynecology, Chungnam National University Hospital) and cervical scrapes from cancer patients (Department of Obstetrics and Gynecology, Chungnam National University Hospital) were obtained, and the methylation degrees of the *ADCYAP1* gene in the tumor tissues and the normal tissues were measured. Genomic DNA was isolated from each of the cancer and normal cervical scrapes using a QIAamp DNA Mini kit (QIAGEN, USA), and 200 ng of the isolated gDNA was treated with bisulfite using an EZ DNA methylation-Gold kit (Zymo Research, USA). The DNA treated with bisulfite was eluted with 20 µℓ of sterile distilled water and used in pyrosequencing.

20 ng of the gDNA treated with bisulfite was amplified by PCR. A PCR reaction solution (20 ng of DNA treated with bisulfite, 5 µℓ of 10X PCR buffer (Enzynomics, Korea), 5 units of Taq polymerase (Enzynomics, Korea), 4 µℓ of 2.5 mM dNTP (Solgent, Korea), and 2 µℓ of 10 pmole/µℓ of PCR primers) was treated at 95 °C for 5 minutes, and then subjected to PCR under the following conditions: 45 cycles of 40 sec at 95 °C, 45 sec at 60 °C and 40 sec at 72 °C, followed by 5 min at 72 °C. The amplification of the PCR product was confirmed by electrophoresis using 2.0% agarose gel.

The PCR product was subjected to pyrosequencing using PyroGold reagent (Biotage, USA) by a PSQ96MA system (Biotage, USA). After pyrosequencing, the degree of methylation was determined by calculating the methylation index. The methylation index was calculated by determining the average rate of cytosine binding to each CpG island.

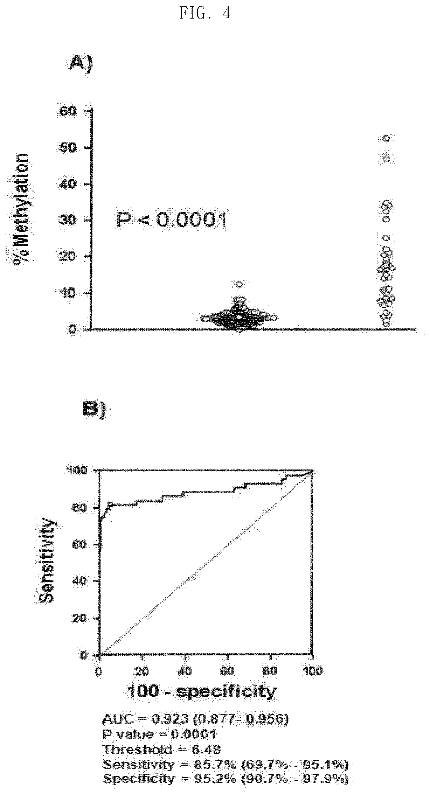

As a result, it was shown that the *ADCYAP1* gene was methylated at very low levels (less than 10%) in the normal cervical scrapes, but methylated at high levels of 10% or higher in most of the cancer tissues (p < 0.0001, FIG. 4A) . In addition, whether cervical cancer can be diagnosed using the methylation degree of the *ADCYAP1* gene was examined by ROC curve analysis using the MedCalc program (Belgium). As a result, the *ADCYAP1* gene showed a very high sensitivity of 85.7% and a very high specificity of 95.2% (see FIG. 4B). Such results indicate that the expression of *ADCYAP1* gene is decreased by methylation in cervical cancer scrape.

### Example 5: Construction of vector for expressing gene that encodes adenylate cyclase activating polypeptide 1 (pituitary)

In order to construct an expression vector for expressing the *ADCYAP1* gene in cells, a 531-bp cDNA clone (SEQ ID NO: 1) comprising a full-length *ADCYAP1* gene was purchased from Invitrogen. To clone the 531-bp nucleotide sequence into the *Hind*III and *Xho*I sites of a pcDNA3 expression vector (Invitrogen, USA), PCR was performed. For PCR amplification, primers of SEQ ID NOS: 7 and 8 were used. The *ADCYAP1* gene was cloned into the *Hind*III and *Xho*I sites of the pcDNA3 expression vector, thereby constructing an *ADCYAP1* gene expression vector (p*ADCYAP1*).
SEQ ID NOS: 7: 5'-ttt aagctt atgaccatgt gtagcggagc-3'
SEQ ID NOS: 8: 5'-ttc ctcgag ctacaaataagctattcggc-3'

### Example 6: Introduction of gene that encodes adenylate cyclase activating polypeptide 1 (pituitary) into cervical cancer cells and measurement of whether the gene inhibits proliferation of cancer cells

In order to examine the effect of the over-expression of the ADCYAPl-encoding gene on the apoptosis of cervical cancer cells, a TUNEL assay was performed.

To perform an apoptosis experiment, the cervical cancer cell line HeLa (KCLB 10002) was seeded into a 4-well chamber at a concentration of 1.75 x 10⁴ cells/well and cultured in a 5% CO₂ incubator (Forma Scientific Inc.) using a DMEM medium (Gibco, USA) containing 10% FBS (Sigma, USA), penicillin (100 units/Mℓ, WelGENE, Korea) and streptomycin (100 units/Mℓ) at 37 °C for 24 hours.

The cultured cervical cancer cells were transfected with the *ADCYAP1* gene expression vector (p*ADCYAP1*) (constructed in Example 5) using a Fugene HD transfection reagent (Roche Applied Science). As a control, pcDNA3 containing no *ADCYAP1* gene was transfected into the cells. The transfected cells were incubated for 48 hours, and then fixed with 4% PFA (paraformaldehyde). Then, 50 µℓ of a TUNEL assay mixture (Roche Applied Science) was added to each well and incubated in a 5% CO₂ incubator at 37 °C for 1 hour. After removal of the reagent, apoptosis of the cells was observed under a fluorescence microscope.

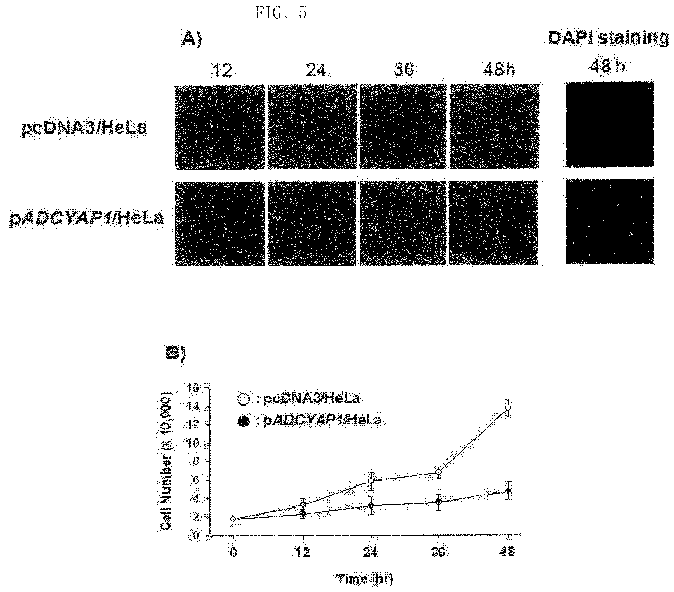

As a result, in the cervical cancer cells introduced with the p*ADCYAP1* expression vector containing the *ADCYAP1* gene, intranuclear DNA fragmentation (apoptosis) significantly increased with the passage of cell culture time (see FIG. 5A). Also, the results of measurement of the cell growth curve after transfection indicated that the growth of the cell line introduced with the p*ADCYAP1* expression vector was significantly inhibited (see FIG. 5B). This suggests that the *ADCYAP1* gene or its protein can induce the apoptosis of cervical cancer cells and can be used as an agent for treating cervical cancer.

### Example 7: Measurement of whether proliferation of cervical cancer cells is inhibited by treatment with peptide fragment of adenylate cyclase activating polypeptide 1 (pituitary)

In order to examine whether the apoptosis of cervical cancer cells is induced when the cervical cancer cells are treated with a peptide fragment of the *ADCYAP1* protein, a WST assay was performed.

To perform an apoptosis test, the cervical cancer cell line HeLa (KCLB 10002) was cultured in a 6-well plate at a concentration of 5 x 10⁴ cells, and then treated with 1 nM or 100 nM of each an *ADCYAP1* (1-27) peptide fragment (GenScript, USA) and an *ADCYAP1* (1-38) peptide fragment (GenScript, USA) in distilled water for 18 hours.

*ADCYAP1* (1-27): HSDGIFTDSYSRYRKQMAVKKYLAAVL-NH₂ (C-terminal NH₂ modified; SEQ ID NO: 9); a peptide fragment having the first 27 amino acid residues from the N-terminus of *ADCYAP1* protein.

*ADCYAP1* (1-38): HSDGIFTDSYSRYRKQMAVKKYLAAVLGKRYKQRVKNK-NH₂ (C-terminal NH₂ modified; SEQ ID NO: 10); a peptide fragment having the first 30 amino acid residues from the N-terminus of *ADCYAP1* protein.

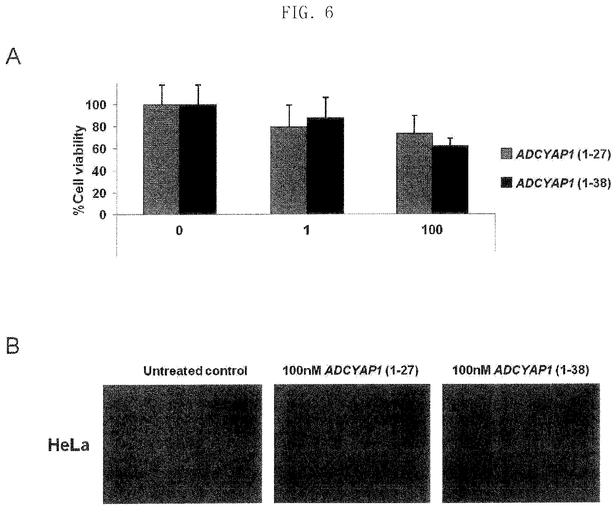

10 µl of WST reagent (Takara, Japan) was added to each of the control group (not treated with the peptide fragment) and the peptide fragment-treated cell groups to induce color development, and the absorbance at 480 nm was measured using a microplate reader. As a result, the absorbance of the cells treated with each of 1 nM of the peptide fragment and 100 nM of the peptide fragment decreased by 20% or more compared to that of the control group (not treated with the peptide fragment), suggesting that the peptide fragment of *ADCYAP1* inhibits the growth of the cervical cancer cell line by 20% or more (see FIG. 6A).

In addition, the cervical cancer cell line HeLa (KCLB 10002) was cultured in a 6-well plate at a concentration of 2.5x10⁵ cells, and then treated with 100 nM of each of the *ADCYAP1* (1-27) peptide fragment and the *ADCYAP1* (1-38) peptide fragment in distilled water for 18 hours. The growth of the cells not treated with the *ADCYAP1* peptide fragment and the cells treated with the *ADCYAP1* peptide fragment was observed under an optical microscope (see FIG. 6B). As a result, the growth of the cervical cancer cells treated with each of the *ADCYAP1* (1-27) peptide fragment and the *ADCYAP1* (1-38) peptide fragment was significantly inhibited.

The above results suggest, that the *ADCYAP1* protein or the peptide fragment of the *ADCYAP1* protein can induce the apoptosis of cervical cancer cells and can be used as an agent for treating cervical cancer.

### INDUSTRIAL APPLICABILITY

As described above, the present invention provides a pharmaceutical composition for inhibiting the proliferation of cervical cancer cells and treating cervical cancer, which contains a recombinant vector comprising a gene of SEQ ID NO: 1 that encodes adenylate cyclase activating polypeptide 1 (pituitary) (*ADCYAP1*), or a fragment of the gene, or adenylate cyclase activating polypeptide 1 of SEQ ID NO: 2, or a fragment.
## Claims

1. A pharmaceutical composition, wherein the composition comprises
(i) a recombinant vector comprising a gene of SEQ ID NO: 1 that encodes adenylate cyclase activating polypeptide 1 (pituitary) (ADCYAP1 ; NM_001099733) or a gene that encodes a fragment of ADCYAP1 comprising a sequence of SEQ ID NO: 9 or 10 as an active ingredient, or
(ii) adenylate cyclase activating polypeptide 1 (pituitary) (ADCYAP1; NM_001099733) of SEQ ID NO: 2, or a fragment of ADCYAP1 comprising a sequence of SEQ ID NO: 9 or 10 as an active ingredient, for use in the treatment of cervical cancer.

## Patentansprüche

1. Pharmazeutische Zusammensetzung, wobei die Zusammensetzung:
(i) einen rekombinanten Vektor, der ein Gen mit SEQ ID NO:1, das Adenylatcyclase-aktivierendes Polypeptid 1 (hypophysär) (ADCYAP1; NM_001099733) codiert, oder ein Gen, das ein Fragment von ADCYAP1, das eine Sequenz von SEQ ID NO:9 oder 10 umfasst, codiert, umfasst, als aktives Ingredienz oder
(ii) Adenylatcyclase-aktivierendes Polypeptid 1 (hypophysär) (ADCYAP1; NM_001099733) mit SEQ ID NO:2 oder ein Fragment von ADCYAP1, das eine Sequenz von SEQ ID NO:9 oder 10 umfasst, als aktives Ingredienz umfasst, zur Verwendung bei der Behandlung von Gebärmutterhalskrebs.

## Revendications

1. Composition pharmaceutique, ladite composition comprenant:
(i) un vecteur recombinant qui comprend un gène de SEQ ID NO: 1 codant pour le polypeptide 1 activant l'adénylate cyclase (hypophysaire) (ADCYAP1; NM_001099733) ou un gène codant pour un fragment de ADCYAP1 qui comprend une séquence de SEQ ID NO: 9 ou 10 comme ingrédient actif, ou
(ii) le polypeptide 1 activant l'adénylate cyclase (hypophysaire) (ADCYAP1; NM_001099733) de SEQ ID NO: 2 ou un fragment de ADCYAP1 qui comprend une séquence de SEQ ID NO: 9 ou 10 comme ingrédient actif pour l'utilisation dans le traitement du cancer du col de l'utérus.


<110> GenomicTree, Inc.
<120> Recombinant Vector Containing a Gene Encoding Adenylate Cyclase Activating Polypeptide 1(pituitary)for Inhibiting Cervival Cancer Cell-Growth and Pharmaceutical Composition for Cervical Cancer Treatment Using the Seme
<130> PP-B1005
<150> KR10-2010-0060196
   <151> 2010-06-24
<160> 10
<170> KopatentIn 1.71
<210> 1
   <211> 531
   <212> DNA
   <213> Homo sapiens
<400> 1
   atgaccatgt gtagcggagc gaggctggcc ctgctggtct atgggataat catgcacagc        60
   agcgtctaca gctcacctgc cgccgccgga ctccggttcc ccgggatcag gccagaggaa       120
   gaggcgtacg gcgaggacgg aaacccgctg ccagacttcg atggctcgga gccgccgggc       180
   gcagggagcc ccgcctccgc gccgcgcgcc gccgccgcct ggtaccgccc ggccgggaga       240
   agagatgtcg cccacgggat ccttaacgag gcctaccgca aagtgctgga ccagctgtcc       300
   gccgggaagc acctgcagtc gctcgtggcc cggggcgtgg gtgggagcct cggcggcggc       360
   gcgggggacg acgcggagcc gctctccaag cgccactcgg acgggatctt cacggacagc       420
   tacagccgct accggaaaca aatggctgtc aagaaatact tggcggccgt cctagggaag       480
   aggtataaac aaagggttaa aaacaaagga cgccgaatag cttatttgta g                531
<210> 2
   <211> 176
   <212> PRT
   <213> Homo sapiens
<400> 2
   Met Thr Met Cys Ser Gly Ala Arg Leu Ala Leu Leu Val Tyr Gly Ile
    1               5                  10                  15
   Ile Met His Ser Ser Val Tyr Ser Ser Pro Ala Ala Ala Gly Leu Arg
               20                  25                  30
   Phe Pro Gly Ile Arg Pro Glu Glu Glu Ala Tyr Gly Glu Asp Gly Asn
           35                  40                  45
   Pro Leu Pro Asp Phe Asp Gly Ser Glu Pro Pro Gly Ala Gly Ser Pro
       50                  55                  60
   Ala Ser Ala Pro Arg Ala Ala Ala Ala Trp Tyr Arg Pro Ala Gly Arg
   65                  70                  75                  80
   Arg Asp Val Ala His Gly Ile Leu Asn Glu Ala Tyr Arg Lys Val Leu
                   85                  90                  95
   Asp Gln Leu Ser Ala Gly Lys His Leu Gln Ser Leu Val Ala Arg Gly
               100                 105                 110
   Val Gly Gly Ser Leu Gly Gly Gly Ala Gly Asp Asp Ala Glu Pro Leu
           115                 120                 125
   Ser Lys Arg His Ser Asp Gly Ile Phe Thr Asp Ser Tyr Ser Arg Tyr
       130                 135                 140
   Arg Lys Gln Met Ala Val Lys Lys Tyr Leu Ala Ala Val Leu Gly Lys
   145                 150                 155                 160
   Arg Tyr Lys Gln Arg Val Lys Asn Lys Gly Arg Arg Ile Ala Tyr Leu
                   165                 170                 175
<210> 3
   <211> 23
   <212> DNA
   <213> Artificial Sequence
<220>
   <223> primer
<400> 3
   gggtggattt ayggttattt tgt 23
<210> 4
   <211> 19
   <212> DNA
   <213> Artificial Sequence
<220>
   <223> primer
<400> 4
   aaattttccc tccttaccc 19
<210> 5
   <211> 19
   <212> DNA
   <213> Artificial Sequence
<220>
   <223> primer
<400> 5
   ggtttttgtt tagatatta 19
<210> 6
   <211> 345
   <212> DNA
   <213> Homo sapiens
<400> 6
   gggtggattt ayggttattt tgttttttyg ygttttattt tatygttttt tttttttttt      60
   ttttgttttt ttttttgygt tttttttttt tygtgttayg tttttttttg gttttgygyg    120
   tttataaatt tttgagtaga ataygagttt yggtaaayga gtttygtagt tttttttgtt    180
   gttttygttg gtttttgygg tttttgttta gatattaayg ttagayggyg atgtttttyg    240
   ggtggtgatt ttagygtagg aatttgaaga agygttttgt tygtygtttt atttggtagt    300
   ttttttggta gygggaggag ttgaagggta agggagggaa aattt                     345
<210> 7
   <211> 29
   <212> DNA
   <213> Artificial Sequence
<220>
   <223> primer
<400> 7
   tttaagctta tgaccatgtg tagcggagc 29
<210> 8
   <211> 29
   <212> DNA
   <213> Artificial Sequence
<220>
   <223> primer
<400> 8
   ttcctcgagc tacaaataag ctattcggc 29
<210> 9
   <211> 27
   <212> PRT
   <213> Homo sapiens
<400> 9
   His Ser Asp Gly Ile Phe Thr Asp Ser Tyr Ser Arg Tyr Arg Lys Gln
    1               5                  10                  15
   Met Ala Val Lys Lys Tyr Leu Ala Ala Val Leu
               20                  25
<210> 10
   <211> 38
   <212> PRT
   <213> Homo sapiens
<400> 10
   His Ser Asp Gly Ile Phe Thr Asp Ser Tyr Ser Arg Tyr Arg Lys Gln
    1               5                  10                  15
   Met Ala Val Lys Lys Tyr Leu Ala Ala Val Leu Gly Lys Arg Tyr Lys
               20                  25                  30
   Gln Arg Val Lys Asn Lys
           35